# EUROPÄISCHE PATENTSCHRIFT

(11) **EP 1 561 097 B1**
(45) Veröffentlichungstag und Bekanntmachung des Hinweises auf die Patenterteilung: **22.08.2007**
(21) Anmeldenummer: 03775336.5
(22) Anmeldetag: 11.11.2003
(51) Int. Cl.: G01N 21/898, G01N 21/89, G01N 33/46, G01B 11/30

(54) **VORRICHTUNG UND VERFAHREN ZUR AUFZEICHNUNG DER OBERFLÄCHENBESCHAFFENHEIT EINES FASERARTIG STRUKTURIERTEN LANGESTRECKTEN GEGENSTANDES**
DEVICE AND METHOD FOR RECORDING THE SURFACE CHARACTERISTICS OF A FIBROUS STRUCTURED ELONGATED OBJECT
DISPOSITIF ET PROCEDE D'ENREGISTREMENT DES CARACTERISTIQUES DE SURFACE D'UN OBJET ALLONGE STRUCTURE FIBREUX

(30) Priorität: 14.11.2002 AT 17172002
(43) Veröffentlichungstag der Anmeldung: 10.08.2005
(73) Patentinhaber: MICROTEC S.r.l., 39042 Bressanone (Bolzano) (IT)
(72) Erfinder: GIUDICEANDREA, Federico, I-39042 Bressanone (IT); BOSCHETTI, Marco, I-38100 Trento (IT)
(74) Vertreter: Ponchiroli, Simone
(86) Internationale Anmeldenummer: PCT/EP2003/012569
(87) Internationale Veröffentlichungsnummer: WO 2004/044566

(56) Entgegenhaltungen:
- CA-A- 2 298 335
- DE-A- 3 805 455
- US-A- 4 188 544
- US-A- 4 260 899
- ASTRAND E ET AL: "A single chip multi-function sensor system for wood inspection" IEEE, 9. Oktober 1994 (1994-10-09), Seiten 300-304, XP010216401
- PATENT ABSTRACTS OF JAPAN vol. 1999, no. 13, 30. November 1999 (1999-11-30) & JP 11 223514 A (NKK CORP), 17. August 1999 (1999-08-17)
- ABBOTT A L ET AL: "Automatic classification of wooden cabinet doors" APPLICATIONS OF COMPUTER VISION, 1994., PROCEEDINGS OF THE SECOND IEEE WORKSHOP ON SARASOTA, FL, USA 5-7 DEC. 1994, LOS ALAMITOS, CA, USA,IEEE COMPUT. SOC, 5. Dezember 1994 (1994-12-05), Seiten 18-25, XP010100113 ISBN: 0-8186-6410-X

## Beschreibung

Die Erfindung betrifft eine Vorrichtung zur Aufzeichnung der Oberflächenbeschaffenheit eines faserartig strukturierten langgestreckten Gegenstandes, eine damit gebildete Oberflächenprüfvorrichtung und ein Verfahren zur Aufzeichnung der Oberflächenbeschaffenheit eines faserartig strukturierten langgestreckten Gegenstandes, entsprechend den Merkmalen in den Oberbegriffen entsprechend den Ansprüchen 1, 8 und 13.

Bei der Holzverarbeitung, wie z.B. in Sägewerken oder der holzverarbeitenden Industrie, ist es aufgrund gestiegener Qualitätsanforderungen erforderlich, Einrichtungen zur Verfügung zu haben, mit deren Hilfe möglichst rasch Qualitätskontrollen bzw. Beurteilungen von Hölzern, wie z.B. Brettern oder Balkenholz, durchgeführt werden können. So ist es z.B. in Sägewerksanlagen erforderlich, defekte Holzstücke bereits nach dem Sägen und noch während des Durchlaufs durch die Anlage in einer effektiven Weise zu identifizieren und diese aussortieren zu können. Qualitätsmindernde Fehlstellen, die zuverlässig identifiziert werden müssen, sind z.B. Spalten, Sprünge, Knoten bzw. Äste oder auch Harzeinschlüsse.

Eine bekannte Methode zur Beurteilung von Holzbalken oder Brettern bedient sich der Aufzeichnung der Oberflächenbeschaffenheit mit Hilfe des sogenannten Tracheideffekts. Dieser Effekt besteht darin, dass beim Auftreffen eines Lichtstrahls auf eine Oberfläche eines faserartig strukturierten Gegenstands das Licht in einem Oberflächenbereich an den Fasern eine mehrfache Streuung erfährt, wobei diese Mehrfachstreuungen in der Faserrichtung begünstigt werden und so der Auftreffpunkt des Lichtstrahls auf der Oberfläche in Faserrichtung verbreitert erscheint. Da die Faserrichtung von Brettern weitestgehend parallel zu deren Längserstreckung ausgerichtet ist, wird üblicherweise ein aufgeweiteter Laserstrahl so auf das zu untersuchende Brett aufgestrahlt, dass er darauf eine Lichtspur erzeugt, die senkrecht zur Längserstreckung des Holzstücks und somit auch senkrecht zur vorherrschenden Faserrichtung ausgerichtet ist. Ist die Faserstruktur des Holzstücks über die gesamte Länge der Lichtspur gleichförmig, so erscheint die linienförmige Lichtspur auch über deren gesamte Länge gleichförmig verbreitert. Treten allerdings an irgend einer Stelle Fehlstellen auf, so sind diese durch eine unterschiedliche, zumeist geringere Verbreiterung der Lichtspur erkennbar. Es ist bekannt, die Bilder der Lichtspuren durch optoelektronische Einrichtungen zu erfassen und während des Durchlaufs der Holzstücke mit einer Transportrichtung in Richtung ihrer Längserstreckung eine Folge von Bildern der Lichtspuren aufzuzeichnen und auf diese Weise eine gesamte Seite eines Holzstücks zu erfassen. Die Folge der Bilder der Lichtspuren kann eiektronisch zu einem Gesamtbild zusammengefügt werden, wobei Fehlstellen bzw. Fehler in der Faserstruktur sich besonders deutlich hervorheben.

Eine Anordnung und Methode zum Nachweis von Fehlstellen in Balkenholz ist z.B. in der WO 95/24636 A1 beschrieben. Es werden dabei eine oder mehrere lineare Lichtquellen und ein Sensor zur Detektion des gestreuten bzw. reflektierten Lichts verwendet. Das Sichtfeld des Sensors ist dabei so eingestellt, dass von diesem nur das Licht das aus einem Bereich, der an den von der Lichtquelle beleuchteten Bereich unmittelbar angrenzt, empfangen werden kann. In einer weiteren Ausführungsform wiederum wird vom Detektor Licht aus dem unmittelbar von den Lichtquellen beleuchteten Bereichen als auch den diesen benachbarten Bereichen empfangen und analysiert, wobei die entsprechenden Messsignale des Sensors in weiterer Folge durch Signalverarbeitungs- bzw. Bildverarbeitungseinrichtungen zur Identifizierung von Fehlstellen an der Holzoberfläche verwendet werden.

Bei modernen Sägewerksanlagen mit hoher Leistung wird mehr und mehr dazu übergegangen, die Rundhölzer, Holzbalken als auch Bretter während der Bearbeitung in der Anlage relativ zur Transportrichtung nicht in ihrer Längslage sondern in Querlage zu transportieren. Während beim Transport in Längsrichtung, wo auch die vorherrschende Faserrichtung in Transportrichtung ausgerichtet ist, mit einer Lichtquelle, die eine Lichtspur auf dem Holzbalken erzeugt, die quer zur Transportrichtung und damit auch quer zur vorherrschenden Faserrichtung ausgerichtet ist, sehr einfach während des Durchlaufs des Holzstücks eine gesamte Seite eines Holzbalkens erfasst werden kann, ist die Aufzeichnung der Oberflächenbeschaffenheit unter Verwendung des Tracheideffekts beim Durchlauf der Holzstücke in Querrichtung in der konventionellen Weise nicht möglich. Mit einer ebenfalls quer zur Transportrichtung ausgerichteten Lichtspur liegt dann nämlich die durch den Tracheideffekts hervorgerufene Verbreiterung im Bereich der Lichtspur selbst und ist damit nicht erkennbar. Wegen des üblicherweise großen Verhältnisses zwischen Längen- und Querschnittsabmessungen von Holzstücken ist es dann aber auch nicht möglich, mit einem einzigen System aus Lichtquelle und Detektor, die gesamte Länge eines Holzstücks auf einmal zu erfassen. Da moderne Holzverarbeitungsanlagen mit sehr hohen Durchlaufgeschwindigkeiten arbeiten, stellt aber auch ein System bei der der Holzbalken bzw. das Holzstück angehalten wird und durch ein in Querrichtung bewegbares System von Lichtquelle und Sensor die Oberfläche erfasst wird, keine praktikable Alternative dar.

Eine Vorichtung bei der Holzbalken als auch Bretter während der Aufzeichnung in der Anlage relativ zur Transportrichtung nicht in ihrer Längslage sondern in Querlage transportiert werden ist aus DE 38 05 455 A1 bekannt.

Ausgehend aus dieser bekannten Vorrichtung, deren Merkmale die des Oberbegriffes des Anspruches 1 entsprechen, ist es Aufgabe der Erfindung eine weitere

Vorrichtung bzw. ein Verfahren zu schaffen, mit dem es möglich ist, die Oberflächenbeschaffenheit eines faserartig strukturierten langgestreckten Gegenstandes auch bei einem Transport in einer Lage, wobei die Längserstreckung des Gegenstandes bzw. die bevorzugte Faserrichtung quer zur Transportrichtung ausgerichtet ist, erfasst werden kann.

Die Aufgabe der Erfindung wird durch die Vorrichtung zur Aufzeichnung der Oberflächenbeschaffenheit eines faserartig strukturierten langgestreckten Gegenstandes entsprechend den Merkmalen im Kennzeichen des Anspruchs 1 gelöst. Der Vorteil dieser Vorrichtung liegt dabei darin, dass damit Oberflächenprüfvorrichtungen aufgebaut werden können, mit denen es möglich ist, großflächig als auch quasi kontinuierlich ebene Oberflächen von Gegenständen mit einer bevorzugten Faserrichtung parallel zur Längserstreckung des Gegenstandes zu untersuchen, wobei der Gegenstand in einer Lage transportiert wird, in der die Längserstreckung quer zur Transportrichtung ausgerichtet ist. Dadurch wird insbesondere eine wesentliche Steigerung der Leistungsfähigkeit einer so aufgebauten Oberflächenprüfvorrichtung gegenüber konventionellen Prüfvorrichtungen erreicht.

Durch die ebene Ausbildung der Lichtstrahlenbündel gemäß Anspruch 2 wird der Vorteil erzielt, dass über die gesamte Länge der von den Lichtstrahlenbündeln erzeugten Lichtspuren gleiche Bedingungen herrschen, d.h. der Winkel zwischen der Lichtspur und der bevorzugten Faserrichtung gleich groß ist. Von Vorteil ist aber auch, dass ebene Lichtstrahlenbündel einfacher erzeugt werden können.

Durch die Weiterbildung der Vorrichtung gemäß Anspruch 3 wird der Vorteil erzielt, dass durch die zusätzliche Verwendung eines das Gesichtsfeld der Kamera in einer Richtung vergrößernden Optikelements erreicht werden kann, dass der gesamte Bereich der mit Lichtspuren bestrahlten Oberfläche weitestgehend bildfüllend in der Kamera abgebildet werden kann.

Die Ausbildung der Vorrichtung gemäß Anspruch 4, in dem als Kamera eine CMOS-Kamera verwendet wird, hat den Vorteil, dass bei derartigen Kameras sehr hohe Geschwindigkeiten für das Auslesen der Bildinformation möglich sind.

Durch die Weiterbildung der Vorrichtung gemäß Anspruch 5 wird der Vorteil erzielt, dass nur Bildinformationen aus für die Prüfung relevanten Bereichen der Oberfläche übertragen werden und so die Leistungsfähigkeit der Bildverarbeitung deutlich vergrößert werden kann.

Die Weiterbildung der Vorrichtung gemäß Anspruch 6, in dem die Lichtquellen durch Laser ausgebildet sind, bietet den Vorteil, dass dadurch besonders scharfe bzw. dünne Lichtspuren auf der Oberfläche des zu untersuchenden Gegenstandes erzeugt werden können und somit die durch den Tracheideffekt bewirkte unterschiedliche Linienverbreiterung besonders deutlich erkennbar wird.

Von Vorteil ist auch die Ausbildung der Kamera mit einem Filter gemäß Anspruch 7, da dadurch störende Fremdlichteinflüsse weitestgehend vermieden werden können.

Eigenständig wird die Aufgabe der Erfindung auch durch eine Oberflächenprüfvorrichtung entsprechend den Merkmalen des Kennzeichenteils des Anspruch 8 gelöst. Vorteilhaft ist dabei, dass mit einer derartigen Oberflächenprüfvorrichtung Stückholz, wie Bretter oder Balken, während ihres Transports in einer Lage, in der die Längserstreckung der Gegenstände quer zur Transportrichtung ausgerichtet ist, untersucht werden können und pro Zeiteinheit eine wesentlich größere Anzahl von Gegenständen geprüft werden kann.

Durch die Ausbildung der Oberflächenprüfvorrichtung gemäß Anspruch 9 wird in vorteilhafter Weise erreicht, dass unterschiedliche Lichtstrahlen von einzelnen Lichtstrahlenbündeln weitestgehend unter dem gleichen Winkel relativ zur Oberfläche des Gegenstands auftreffen. Dadurch können möglichst gleiche Bedingungen bzw. Lichtverhältnisse über die Länge einer Lichtspur gewährleistet werden.

Die Weiterbildung der Oberflächenprüfvorrichtung gemäß Anspruch 10 in dem einander benachbarte Lichtspuren bezüglich der Querrichtung zumindest lückenlos aneinander anschließen hat den Vorteil, dass die Oberflächenbeschaffenheit der gesamten Oberfläche aufgezeichnet werden kann.

Durch die Ausstattung der Oberflächenprüfvorrichtung mit einer Steuereinrichtung und einer Auswerteeinheit gemäß Anspruch 11 wird erreicht, dass der Betrieb der Aufzeichnung der Bildinformation als auch die Erstellung eines Gesamtbildes der zu untersuchenden Oberfläche des Gegenstandes automatisiert erfolgen kann.

Vorteilhaft ist auch die Weiterbildung der Oberflächenprüfvorrichtung gemäß Anspruch 12, in dem eine Vorrichtung zur Messung der Distanz angeordnet wird, da dadurch die Bilder der Lichtspuren eindeutig mit den jeweiligen Positionen des Gegenstands korreliert werden können und so die Erzeugung von unverzerrten Gesamtbildern aus den Einzelbildern der Lichtspuren möglich wird.

Die Aufgabe der Erfindung wird eigenständig auch durch das Verfahren entsprechend den Merkmalen im Kennzeichenteil des Anspruches 13 gelöst. Vorteilhaft ist dabei, dass mit diesem Verfahren, bei dem die Bretter bzw. Balken in einer Lage, bei der die Längserstreckung des Gegenstands quer zur Transportrichtung ausgerichtet ist, untersucht werden, eine wesentlich höhere Leistungsfähigkeit erreicht werden kann.

Von Vorteil ist auch die Durchführung des Verfahrens gemäß Anspruch 14, da dadurch die Oberfläche des Gegenstandes unverzerrt dargestellt werden kann. Da das Gesamtbild der Oberfläche des Gegenstands aus einzelnen Bilder der Lichtspuren bei unterschiedlichen Positionen während der Bewegung des Gegenstandes gebildet wird, würde eine dabei erfolgende Rotation des Gegenstandes naturgemäß zu einer entsprechenden Verzerrung in dem Gesamtbild führen.

Zur Beurteilung der Qualität von Brettern bzw. Holzbalken ist es wünschenswert, die gesamte Oberfläche zu untersuchen. Dies kann erreicht werden, indem das Verfahren gemäß Anspruch 15 durchgeführt wird, wobei die Lichtstrahlenbündel so angeordnet werden, dass sie bezüglich der Querrichtung zumindest lückenlos aneinander anschließen.

Die Verwendung einer CMOS-Kamera gemäß Anspruch 16 bei der Durchführung des Verfahrens hat den Vorteil, dass das Auslesen der Bildinformation mit besonders hohen Geschwindigkeiten erfolgen kann.

Vorteilhaft sind auch die Weiterbildungen des Verfahrens gemäß den Ansprüchen 17 und 18, da dadurch die Leistungsfähigkeit bzw. die Durchlaufgeschwindigkeit der zu untersuchenden Gegenstände weiter gesteigert werden kann.

Vorteilhaft ist schließlich auch die Weiterbildung des Verfahrens gemäß Anspruch 19, indem durch die Auswerteeinheit aus der Folge der Bilder der Lichtspuren ein Bild der Oberfläche des Gegenstandes erzeugt wird, da dadurch ein Gesamtbild der Oberfläche zur Verfügung gestellt werden kann, aus dem die Qualität des untersuchten Gegenstandes wesentlich besser ist, weil gesamthaft beurteilt werden kann.

Zum besseren Verständnis der Erfindung wird diese anhand der nachfolgenden Figuren näher erläutert.

Es zeigen in schematisch vereinfachter Darstellung:
- Fig. 1: eine Oberflächenprüfvorrichtung mit einer Vorrichtung zur Aufzeichnungen der Oberflächenbeschaffenheit eines Gegenstandes;
- Fig. 2: ein weiteres Ausführungsbeispiel einer Oberflächenprüfvorrichtung mit zwei Vorrichtungen zur Aufzeichnung der Oberflächenbeschaffenheit.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen.

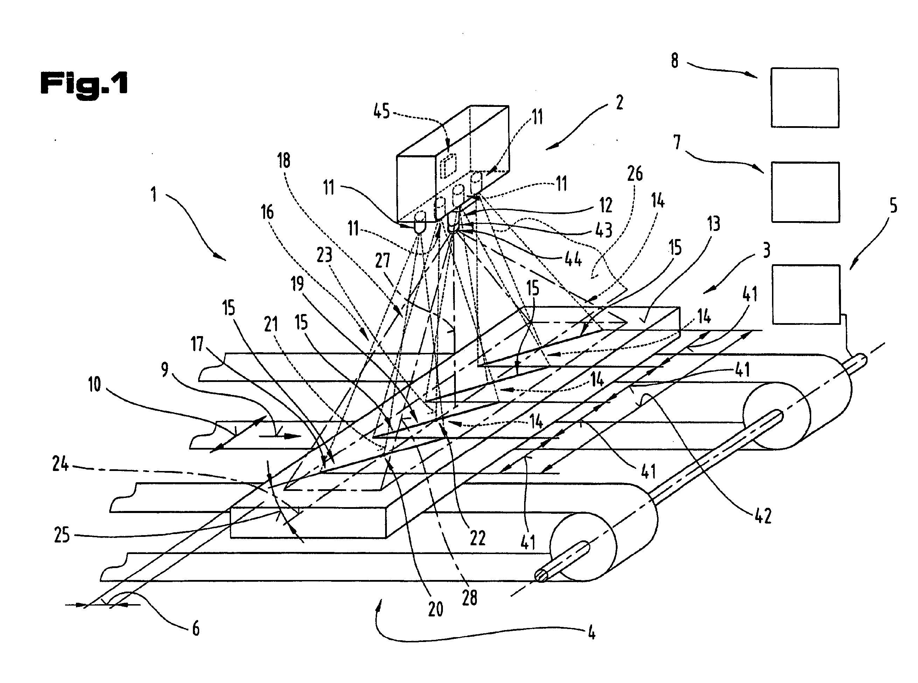

Die Fig. 1 zeigt eine Oberflächenprüfvorrichtung 1 bestehend aus einer Vorrichtung 2 zur Aufzeichnung der Oberflächenbeschaffenheit eines faserartig strukturierten langgestreckten Gegenstands 3 und einer Transporteinrichtung 4. Die Oberflächenprüfvorrichtung 1 verfügt weiters über eine Vorrichtung 5 zur Messung einer Distanz 6, einer Auswerteeinheit 7 und einer Steuereinrichtung 8.

Durch die Transporteinrichtung 4 wird der Gegenstand 3 in Transportrichtung 9 unter der örtlich feststehenden Vorrichtung 2 bewegt. Dabei ist vorgesehen, dass sich der Gegenstand 3 während des Transports in einer Lage befindet, wobei eine Längserstreckung des Gegenstands 3 bezüglich einer zur Transportrichtung 9 senkrechten Querrichtung 10 zumindest annähernd parallel ist.

Die Vorrichtung 2 umfasst vier Lichtquellen 11 und eine Kamera 12. Durch die Lichtquellen 11 wird jeweils ein auf eine Oberfläche 13 des Gegenstands 3 gerichtetes Lichtstrahlenbündel 14 erzeugt. Die Lichtquellen 11 sind dabei so ausgebildet, dass flächenförmige Lichtstrahlenbündel 14 erzeugt werden, wodurch auf der Oberfläche 13 des Gegenstandes 3 linienförmige Lichtspuren 15 entstehen. Die Lichtquellen 11 sind bevorzugt durch einen Laser ausgebildet, wobei die von dieser Lichtquelle 11 emittierten Lichtstrahlen zu einem ebenen Lichtstrahlenbündel 14 aufgefächert sind, so dass im Falle einer ebenen Oberfläche 13 auf dieser Oberfläche 13 eine gerade Lichtspur 15 entsteht. Erfindungsgemäß sind die durch die Lichtquellen 11 der Vorrichtung 2 erzeugten Lichtstrahlenbündel 14 so ausgerichtet, dass die von diesen auf der Oberfläche 13 des Gegenstands 3 erzeugten Lichtspuren 15 bezüglich der Querrichtung 10 längs der eigenen ganzen Länge schräg ausgerichtet sind, wie aus den Figuren ersichtlich ist. Dabei sind zwei einander jeweils benachbarte Lichtstrahlenbündel 15 vorzugsweise in einer Weise angeordnet, dass diese in einer bezüglich der Querrichtung 10 zumindest annähernd parallelen Richtung aufeinander folgen.

Die Vorrichtung 2 zur Aufzeichnung der Oberflächenbeschaffenheit eines faserartig strukturierten langgestreckten Gegenstandes 3 stellt eine für sich eigenständige Einheit dar und die Verhältnisse der räumlichen Anordnung der Lichtstrahlenbündel 14 können auch ohne Bezugnahme auf die Transportrichtung 9 bzw. die Querrichtung 10 der Transporteinrichtung 4 angegeben werden. Demnach wird beim Auftreffen der Lichtstrahlenbündel 14 auf die Oberfläche 13 des Gegenstands 3 durch ein erstes Lichtstrahlenbündel 16 eine erste Lichtspur 17 und durch ein zweites Lichtstrahlenbündel 18 eine zweite Lichtspur 19 erzeugt. Durch einen ersten Auftreffpunkt 20 eines ersten mittleren Lichtstrahls 21 des ersten Lichtstrahlenbündels 16 und einen zweiten Auftreffpunkt 22 eines zweiten mittleren Lichtstrahls 23 des zweiten Lichtstrahlenbündels 18 ist eine Gerade 24 bestimmt. Die Lichtstrahlenbündel 14, 16, 18 sind nunmehr so angeordnet, dass die Lichtspuren 15, 17, 19 bezüglich der Geraden 24 schräg ausgerichtet sind und mit der Geraden 24 einen Winkel 25 von ca. 45° bilden.

Bezüglich der Oberflächenprüfvorrichtung 1 bzw. der Transporteinrichtung 4 ist die Vorrichtung 2 so angeordnet, dass von dieser erzeugte Strahlenbündel 14, 16, 18 bzw. Lichtspuren 15, 17, 19 auf der Oberfläche 13 des Gegenstands 3 längs der eigenen ganzen Länge bezüglich der Querrichtung 10 mit einem Winkel 25 von ca. 45° schräg ausgerichtet sind, wie aus den Figuren ersichtlich ist. Dabei ist außerdem die Lichteinfallsrichtung der Lichtstrahlenbündel 14, 16, 18 so gewählt, dass diese bezüglich der Transportrichtung 9 zumindest annähernd senkrecht ausgerichtet ist. Das heißt eine Ebene 26, die durch den ersten Auftreffpunkt 20 und den zweiten Auftreffpunkt 22 der Lichtstrahlen 21 bzw. 23 und durch eine mittlere Position der Vorrichtung 2 bzw. der die Lichtstrahlenbündel 14, 16, 18 erzeugenden Lichtquellen 11 bestimmt ist, zumindest annähernd senkrecht bezüglich der Transportrichtung 9 ausgerichtet ist. Entsprechend ist auch eine optische Achse **27,** zumindest annähernd senkrecht bezüglich der Transportrichtung 9 als auch bezüglich der Querrichtung 10 ausgerichtet. Die Lichtstrahlenbündel 14, 16, 18 sind zueinander divergent und bezüglich der optischen Achse 27 der Kamera 12 im Wesentlichen symmetrisch angeordnet. Ein Gesichtsfeld 28 der Kamera 12 kann demgemäss so eingestellt werden, dass die Lichtspuren 15, 17, 19 in beiden Richtungen bezüglich der Querrichtung 10 bilderfüllend abgebildet werden.

Da die vorherrschende Faserrichtung entsprechend der Längserstreckung des Gegenstands 3 bzw. parallel zur Querrichtung 10 ausgerichtet ist, kann durch die Schrägstellung der Lichtstrahlenbündel 14, 16, 18 zweierlei erreicht werden. Die durch den Tracheideffekt bewirkte Verbreiterung der Lichtspuren 15, 17, 19 in Querrichtung 10 bzw. der vorherrschenden Faserrichtung bleibt erstens ausreichend gut sichtbar bzw. detektierbar während zweitens durch die Lichtspuren 15, 17, 19 jeweils ein ausreichend großer, in Querrichtung 10 gerichteter Bereich 41 der Oberfläche 13 überstrichen werden kann. Mit parallel zur Querrichtung 10 ausgerichteten Lichtspuren 15, 17, 19 könnten zwar die Bereiche 41 vergrößert werden, die Verbreiterung der Lichtspuren 15, 17, 19 die ebenfalls in Querrichtung 10 gerichtet ist, wäre aber in diesem Fall nicht mehr sichtbar. Im Ausführungsbeispiel gemäß Fig. 1 ist vorgesehen, dass die Bereiche 41 der Lichtspuren 15, 17, 19 lückenlos aneinander anschließen und dass jeweils zwei einander benachbarte Bereiche 41 bzw, jeweils zwei einander benachbarte Lichtspuren 15 einander bezüglich der Querrichtung 10 überlappen bzw. bezüglich der Querrichtung 10 lückenlos aneinander anschließen. Auf diese Weise kann erreicht werden, dass ein aus mehreren Bereichen 41 zusammenhängender Bereich 42 durch die Vorrichtung 2 erfasst werden kann. Durch die in der beschriebenen Weise schräg angeordneten Lichtstrahlenbündel 14, 16, 18 bzw. Lichtspuren 15, 17, 19 ist es somit möglich, sowohl die Verbreiterung der Lichtspuren 15, 17, 19 sichtbar zu machen als auch gleichzeitig mit einer begrenzten Anzahl von Lichtquellen 11 einen Bereich 42 der Oberfläche 13 über seine gesamte Länge lückenlos zu erfassen. Die Anordnung der Vorrichtung 2 der Oberflächenprüfvorrichtung 1 bzw. die Ausrichtung der Lichtstrahlenbündel 14 ist in der Weise vorgesehen, dass der Bereich 42 einer Länge von etwa 1 bis 1,5 m entspricht.

Es ist weiters möglich, eine unterschiedliche Anzahl von Lichtquellen 11 vorzusehen, es erweist sich allerdings als günstig, vier Lichtquellen 11 und eine Kamera 12 in einer Vorrichtung 2 miteinander zu kombinieren. Dabei kann auch vorgesehen sein, dass die Kamera 12 mit einem Optikelement 43 ausgebildet ist, durch das das Gesichtsfeld 28 der Kamera 12 in Richtung der Geraden 24 bzw. parallel zur Querrichtung 10 durch das Optikelement 43 vergrößert ist. Dadurch kann erreicht werden, dass die Lichtspuren 15,17, 19 sowohl bezüglich der Querrichtung 10 als auch bezüglich der Transportrichtung 9 bildfüllend in der Kamera 12 abgebildet werden. Das Optikelement 43 kann z.B. durch einen in nur eine Richtung konvex gekrümmten Parabolspiegel oder ein in nur eine Richtung zerstreuend wirkendes Linsensystem ausgebildet sein. Da vorgesehen ist, dass durch die Kamera 12 der gesamte Bereich 42 durch das Gesichtsfeld 28 erfasst wird, ist durch die Verwendung des Optikelements 43 die optische Auflösung bezüglich der Transportrichtung 9 gegenüber der optischen Auflösung bezüglich der Querrichtung 10 vergrößert. Weiters kann vorgesehen sein, dass die Kamera 12 mit einem Filter 44 ausgebildet ist, der weitestgehend Licht mit Ausnahme des Lichts der als Lichtquellen 11 verwendeten Laser zurückhält. Dadurch können störende Fremdlichteinflüsse weitestgehend vermieden werden.

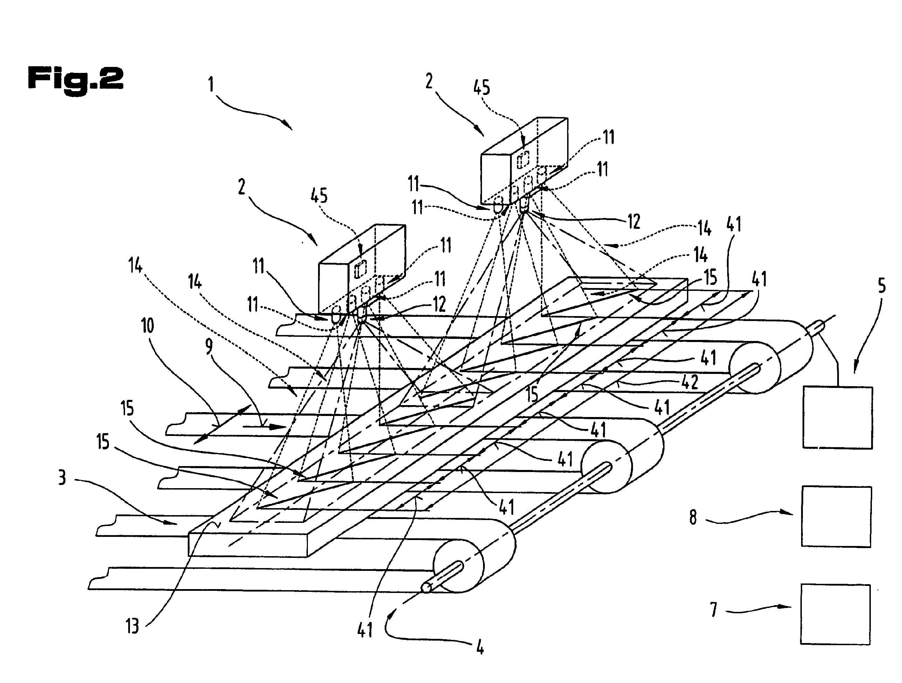

In der Fig. 2 ist eine weitere und gegebenenfalls für sich eigenständige Ausführungsform der Oberflächenprüfvorrichtung gezeigt, wobei wiederum für gleiche Teile gleiche Bezugszeichen wie in der vorangegangenen Fig. 1 verwendet werden.

Fig. 2 zeigt ein weiteres Ausführungsbeispiel einer Oberflächenprüfvorrichtung 1 mit zwei Vorrichtungen 2. In der schon beschriebenen Weise werden durch die Lichtquellen 11 bzw. die entsprechenden Lichtstrahlenbündel 14 auf der Oberfläche 13 des Gegenstandes 3 linienförmige Lichtspuren 15 erzeugt. Während der durch die Transporteinrichtung 4 in Transportrichtung 9 bewirkten Bewegung des Gegenstands 3 wird durch die Lichtspuren 15 jeweils ein in Querrichtung 10 gerichteter Bereich 41 überstrichen. Dabei ist vorgesehen, dass jeweils zwei einander benachbarte Bereiche 41 bzw. jeweils zwei einander benachbarte Lichtspuren 15 einer Vorrichtung 2 einander bezüglich der Querrichtung 10 überlappen bzw. bezüglich der Querrichtung 10 lückenlos aneinander anschließen. Die beiden Vorrichtungen 2 sind bezüglich der Querrichtung 10 gegeneinander versetzt angeordnet, wobei die aus den jeweiligen Bereichen 41 zusammengesetzten Bereiche 42 der beiden Vorrichtungen 2 einander bezüglich der Querrichtung 10 überlappen bzw. lückenlos aneinander anschließen. Auf diese Weise kann die Oberfläche 13 des Gegenstandes 3 über den gesamten Bereich der zusammenhängenden Bereiche 41 durch die Oberflächenprüfvorrichtung 1 erfasst bzw. geprüft werden.

Es ist selbstverständlich möglich, durch aneinander reihen von mehreren Vorrichtungen 2 Oberflächenprüfvorrichtungen 1 für langgestreckte Gegenstände 3 beliebiger Länge zu schaffen.

Mit der Oberflächenprüfvorrichtung 1 gemäß Fig. 1 bzw. gemäß Fig. 2 ist es möglich, ein Verfahren zur Aufzeichnung der Oberflächenbeschaffenheit eines faserartig strukturierten, langgestreckten Gegenstandes 3 auszuführen. Die Darstellung gemäß den Fig. 1 und 2 zeigt die Stellung des Gegenstandes 3 zu einem bestimmten Zeitpunkt während seiner Bewegung durch die Oberflächenprüfvorrichtung 1. Von dem Bild, das von der Kamera 12 zu dem entsprechenden Zeitpunkt aufgezeichnet wird, sind nur jene Bildinformationen relevant, die von denjenigen Bereichen der Oberfläche 13 des Gegenstandes 3 stammen, die gerade von den Lichtspuren 15 berührt werden. Durch die Steuereinrichtung 8 wird veranlasst, dass zu aufeinander folgenden Zeitpunkten von der Kamera 12 jeweils ein Bild der Lichtspuren 15 und von der Vorrichtung 5 die Distanz 6 entsprechend der augenblicklichen Position des Gegenstandes 3 aufgezeichnet wird. Die Distanz 6 ist in Fig. 1 symbolhaft dargestellt und steht für den Abstand zwischen zwei aufeinander folgenden Positionen des Gegenstands 3 zu zwei Zeitpunkten, an denen von der Kamera 12 jeweils ein Bild aufgezeichnet wird. Die Bewegung des Gegenstands 3 durch die Transporteinrichtung 4 erfolgt vorteilhafterweise kontinuierlich mit einer gleichförmigen Geschwindigkeit. Während somit der Gegenstand 3 durch die Transporteinrichtung 4 in Transportrichtung 9 weiterbewegt wird, wird eine Folge von Bildern der Lichtspuren 15 und eine Folge von mit diesen Bildern korrelierten Distanzen 6 aufgezeichnet. Die Bildinformationen bzw. Bildpunkte und die Werte der Distanz 6 werden in die Auswerteeinheit 7 übertragen. Auf der Basis der Information über die jeweilige Position des Gegenstandes 3, die durch die Aufzeichnung der Folge der Distanzen 6 verfügbar ist, ist es möglich, durch die Auswerteeinheit aus der Folge der Bilder der Lichtspuren 15 ein Bild der Oberfläche des Gegenstands zu erzeugen. Aus diesem so erhaltenen Bild ist sodann die Oberflächenbeschaffenheit, d.h. die Gleichförmigkeit oder Ungleichförmigkeit der faserartigen Struktur der Oberfläche 13 des Gegenstandes 3 erkennbar.

Bedingt durch die sehr hohen Durchlaufgeschwindigkeiten moderner Sägewerks- bzw. Holzverarbeitungsanlagen mit bis zu 200 Holzstücken pro Minute ist vorgesehen, dass die Vorrichtung 2 der Oberflächenprüfvorrichtung 1 mit einer Kamera 12 mit besonders kurzer Belichtungszeit ausgestattet ist. Es wird daher bevorzugt eine elektronische Kamera 12, insbesondere eine CMOS-Kamera verwendet, mit der mindestens 1.000 Bilder pro Sekunde aufgezeichnet werden können. Bevorzugt werden mit der CMOS-Kamera etwa 1.500 Bilder pro Sekunde aufgezeichnet. Um die von der Kamera 12 zur Auswerteeinheit 7 zu übertragenden Datenmengen möglichst gering zu halten, kann weiters vorgesehen werden, dass von der Kamera nur Bildpunkte von relevanten Bereichen, d.h. von Bereichen in denen Lichtspuren 15 vorhanden bzw. abgebildet sind, an die Auswerteeinheit 7 übertragen werden. Dies kann dadurch erreicht werden, dass die Kamera 12 über einen entsprechenden elektronischen Bauteil 45 verfügt, der eine Vorbearbeitung der aufgezeichneten Bildpunkte durchführt.

In den Fig. 1 und 2 ist der Gegenstand 3 quaderförmig ausgebildet dargestellt und folglich die durch die Vorrichtung 2 bzw. die Lichtspuren 15 überstrichene Oberfläche 13 ebenflächig ausgebildet. Dies ist jedoch keineswegs zwingend erforderlich und es ist auch möglich, dass Gegenstände 3 mit gekrümmter Oberfläche 13 durch die Vorrichtung 2 bzw. die Oberflächenprüfvorrichtung 1 untersucht werden können. Ebenso kann es vorkommen, dass der Gegenstand während seiner Bewegung durch die Oberflächenprüfvorrichtung 1, wenn auch unbeabsichtigt, eine Rotation bezüglich der Querrichtung 10 bzw. bezüglich seiner Längserstreckung ausführt. Zwar führt dies zu einer verzerrten Darstellung der Oberfläche 13 des Gegenstands 3, es ist aber dennoch auch in einem solchen Fall aus den erhaltenen Bildinformationen eine Beurteilung der Faserstruktur möglich. Von Vorteil ist es jedenfalls, dass die Transporteinrichtung 4 derart ausgebildet ist, dass der Gegenstand 3 während seiner Bewegung in rein translatorischer Weise weiterbewegt wird. Dadurch wird erreicht, dass das in der Auswerteeinheit 7 der Oberflächenprüfvorrichtung 1 erzeugte Gesamtbild der Oberfläche 13 unverzerrt erscheint.

Der Ordnung halber sei abschließend darauf hingewiesen, dass zum besseren Verständnis des Aufbaus der Vorrichtung zur Aufzeichnung der Oberflächenbeschaffenheit bzw. der Oberflächenprüfvorrichtung diese bzw. deren Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

## Patentansprüche

1. Vorrichtung (2) zur Aufzeichnung der Oberflächenbeschaffenheit eines faserartig strukturierten langgestreckten Gegenstands (3)
mit einer bevorzugten Faserrichtung parallel zur Längserstreckung des Gegenstandes (3),
wobei der Gegenstand (3) in einer Lage transportiert wird, in der die Längserstreckung in eine Querrichtung (10) quer zur Transportrichtung ausgerichtet ist, umfassend zumindest eine lichtstrahlenemittierende Lichtquelle (11) und zumindest eine Kamera (12) zur Aufzeichnung von von dem Gegenstand (3) reflektierten Lichtstrahlen,
wobei die eine Lichtquelle (11) bzw. die Lichtquellen (11) zur Erzeugung von zumindest zwei, auf eine Oberfläche (13) des Gegenstands (3) gerichteten, flächenförmigen Lichtstrahlenbündeln (14) ausgebildet ist bzw. sind und wobei ein erstes Lichtstrahlenbündel (16) und ein zweites Lichtstrahlenbündel (18) so ausgerichtet ist, dass beim Auftreffen der Lichtstrahlenbündel (14) auf eine selbe ebene Oberfläche (13) des Gegenstands (3), parallel zu der genannten Querrichtung (10) und der genannten Transportrichtung, die zwei Lichtstrahlenbündel (16, 18) eine erste Lichtspur (17) und eine zweite Lichtspur (19) auf der Oberfläche (13) erzeugen, **dadurch gekennzeichnet dass** die erzeugten Lichtspuren (17, 19) bezüglich der Querrichtung (10) längs der eigenen ganzen Länge schräg ausgerichtet sind.

2. Vorrichtung (2) nach Anspruch 1**, dadurch gekennzeichnet, dass** die Lichtstrahlenbündel (14) jeweils eben ausgebildet sind.

3. Vorrichtung (2) Anspruch 1 oder 2, **dadurch gekennzeichnet, dass** die Kamera (12) mit einem Optikelement (43) ausgebildet ist und ein Gesichtsfeld (28) der Kamera (12) in Richtung der durch einander benachbarte Auftreffpunkte (20, 22) der mittleren Lichtstrahlen (21, 23) gebildeten Geraden (24) durch dieses Optikelement (43) vergrößert ist.

4. Vorrichtung (2) nach einem der vorhergehenden Ansprüche, **dadurch gekennzeichnet, dass** die Kamera (12) durch eine CMOS-Kamera ausgebildet ist.

5. Vorrichtung (2) nach einem der vorhergehenden Ansprüche, **dadurch gekennzeichnet, dass** die Kamera (12) mit einem elektronischen Bauteil (45) zur Vorbearbeitung der aufgezeichneten Bildpunkte bzw. Reduktion der zu übertragenden Datenmenge ausgestattet ist.

6. Vorrichtung (2) nach einem der vorhergehenden Ansprüche, **dadurch gekennzeichnet, dass** die Lichtquelle (11) bzw. die Lichtquellen (11) durch Laser ausgebildet ist bzw. sind.

7. Vorrichtung (2) nach Anspruch 6, **dadurch gekennzeichnet, dass** die Kamera (12) mit einem Filter (44) zum Zurückhalten von Licht außer dem Licht der Laser ausgebildet ist.

8. Oberflächenprüfvorrichtung (1) zur Aufzeichnung der Oberflächenbeschaffenheit eines faserartig strukturierten langgestreckten Gegenstands (3), umfassend
eine Transporteinrichtung (4) mit einer Transportrichtung (9) und einer dazu senkrechten Querrichtung (10) zum Transport des Gegenstands (3) in einer bezüglich der Querrichtung (10) zumindest annähernd parallelen Lage einer Längserstreckung des Gegenstands (3) und zumindest eine Vorrichtung (2) zur Aufzeichnung der Oberflächenbeschaffenheit eines faserartig strukturierten langestreckten Gegenstands (3)
mit einer bevorzugten Faserrichtung parallel zur Längserstreckung des Gegenstandes (3),
wobei der Gegenstand (3) in einer Lage transportiert wird, in der die Längserstreckung in eine Querrichtung (10) quer zur Transportrichtung ausgerichtet ist, wobei die genannte Vorrichtung (2) zur Aufzeichnung in Übereinstimmung mit einem beliebigen der vorstehenden Patentansprüche hergestellt ist,
und wobei jeweils zwei einander benachbarte Lichtstrahlenbündel (14), erzeugt durch die genannte wenigstens eine Lichtquelle (11), in einer bezüglich der Querrichtung (10) zumindest annähernd parallelen Richtung aufeinanderfolgen.

9. Oberflächenprüfvorrichtung (1) nach Anspruch 8, **dadurch gekennzeichnet, dass** eine Ebene (26), die durch eine mittlere Position von die Lichtstrahlenbündel (14) erzeugenden Lichtquellen (11) und einen ersten Auftreffpunkt (20) eines ersten mittleren Lichtstrahls (21) eines ersten Lichtstrahlenbündels (16) und einen zweiten Auftreffpunkt (22) eines zweiten mittleren Lichtstrahls (23) eines zweiten Lichtstrahlenbündels (18) bestimmt ist, zumindest annähernd senkrecht bezüglich der Transportrichtung (9) ausgerichtet ist.

10. Oberflächenprüfvorrichtung (1) nach Anspruch 8 oder 9, **dadurch gekennzeichnet, dass** die Lichtstrahlenbündel (14) so angeordnet sind, dass jeweils zwei einander benachbarte Lichtspuren (15) einander bezüglich der Querrichtung (10) überlappen bzw. bezüglich der Querrichtung (10) lückenlos aneinander anschließen.

11. Oberflächenprüfvorrichtung (1) nach einem der Ansprüche 8 bis 10, **dadurch gekennzeichnet, dass** eine Steuereinrichtung (8) und eine Auswerteeinheit (7) angeordnet ist.

12. Oberflächenprüfvorrichtung (1) nach einem der Ansprüche 8 bis 11, **dadurch gekennzeichnet, dass** eine Vorrichtung (5) zur Messung einer Distanz (6), um die der Gegenstand (3) in Transportrichtung (9) weiterbewegt wird, angeordnet ist.

13. Verfahren zur Aufzeichnung der Oberflächenbeschaffenheit eines faserartig strukturierten, langgestreckten Gegenstands (3), bei dem
eine Transporteinrichtung (4) mit einer Transportrichtung (9) und einer dazu senkrechten Querrichtung (10) zum Transport des Gegenstands (3) in einer bezüglich der Querrichtung (10) zumindest annähernd parallelen Lage einer Längserstreckung des Gegenstands (3) angeordnet wird,
zumindest eine Lichtquelle (11) zur Erzeugung von zumindest zwei auf eine Oberfläche (13) des Gegenstands (3) gerichteten, flächenförmigen Lichtstrahlenbündeln (14) angeordnet wird, wobei von dieser erzeugte Lichtstrahlenbündel (14) bzw. von dieser erzeugte Lichtspuren (15) auf der Oberfläche (13) des Gegenstands (3) und jeweils zwei einander benachbarte Lichtstrahlenbündel (14) in einer bezüglich der Querrichtung (10) zumindest annähernd parallelen Richtung aufeinanderfolgen,
eine Kamera (12) zur Aufzeichnung der Lichtspuren (15) angeordnet wird,
eine Vorrichtung (5) zur Messung einer Distanz (6), um die der Gegenstand (3) in Transportrichtung (9) weiterbewegt wird, angeordnet wird,
eine Steuereinrichtung (8) und eine Auswerteeinheit (7) angeordnet wird und
der Gegenstand (3) durch die Transporteinrichtung (4) in Transportrichtung (9) weiterbewegt wird, wobei eine Folge von Bildern der Lichtspuren (15) und eine Folge von mit den Bildern korrelierten Distanzen (6) aufgezeichnet wird, **dadurch gekennzeichnet, dass**
die beiden flachen Lichtstrahlenbündel (14) auf solche Weise erzeugt werden, dass auf einer selben ebenen Oberfläche (13) des Gegenstandes (3), parallel zu der genannten Transportrichtung (9) und zu der genannten Querrichtung (10), Lichtspuren (15) erzeugt werden, die über ihre gesamte eigene Länge, sei es im Verhältnis zu der Querrichtung (10) wie im Verhältnis zu der Transportrichtung (9), schräg ausgerichtet sind.

14. Verfahren nach Anspruch 13, **dadurch gekennzeichnet, dass** der Gegenstand (3) durch eine rein translatorische Bewegung weiterbewegt wird.

15. Verfahren nach Anspruch 13 oder 14, **dadurch gekennzeichnet, dass** die Lichtstrahlenbündel (14) so angeordnet werden, dass jeweils zwei einander benachbarte Lichtspuren (15) einander bezüglich der Querrichtung (10) überlappen bzw. bezüglich der Querrichtung (10) lückenlos aneinander anschließen.

16. Verfahren nach einem der Ansprüche 13 bis 15, **dadurch gekennzeichnet, dass** als Kamera (12) eine elektronische Kamera (12), insbesondere eine CMOS-Kamera, verwendet wird.

17. Verfahren nach einem der Ansprüche 13 bis 16, **dadurch gekennzeichnet, dass** von der Kamera (12) nur Bildpunkte von Bereichen, in denen Lichtspuren (15) vorhanden sind, an die Auswerteeinheit (7) übertragen werden.

18. Verfahren nach einem der Ansprüche 13 bis 17, **dadurch gekennzeichnet, dass** durch die Auswerteeinheit (7) aus der Folge der Bilder der Lichtspuren (15) ein Bild der Oberfläche (13) des Gegenstands (3) erzeugt wird.

## Claims

1. A device (2) for recording the surface characteristics of a fibrous structured elongated object (3) with a direction of the fibres preferably parallel with the longitudinal extension of the object (3), in which the object (3) is transported in a position in which the longitudinal extension is angled in a transversal direction (10) obliquely to the direction of transport, comprising at least one light source (11) emitting rays of light and at least one camera (12) for recording the rays of light reflected by the object (3), in which a light source (11) or light sources (11) is or are structured to generate at least two flat beams of light (14), directed on a surface (13) of the object (3) and in which a first beam of light (16) and a second beam of light (18) is positioned in such a way that at the point of incidence of the beams of light (14) on the same flat surface (13) of the object (3), parallel with the transversal direction (10) and with the direction of transport, the two beams of light (16, 18) generate a first track of light (17) and a second track of light (19) on the surface (13), the device being **characterised in that** the tracks of light (17, 19) generated with reference to the transversal direction (10) are at an oblique angle along the entire length.

2. The device (2) according to claim 1, **characterised in that** the beams of light (14) respectively have a flat structure.

3. The device (2) according to claim 1 or 2, **characterised in that** the camera (12) consists of an optical element (43) and a field of vision (28) of the camera (12) in the direction of the straight lines (24) formed by the points of incidence (20, 22) side by side of the central rays of light (21, 23) is enlarged by this optical element (43).

4. The device (2) according to any of the foregoing claims, **characterised in that** the camera (12) is a CMOS camera.

5. The device (2) according to any of the foregoing claims, **characterised in that** the camera (12) has an electronic component (45) for preparing the recorded image points, that is to say, for reducing the quantity of data to be transferred.

6. The device (2) according to any of the foregoing claims, **characterised in that** the light source (11) or the light sources (11) is or are a laser.

7. The device (2) according to claim 6, **characterised in that** the camera (12) is made with a filter (44) to stop the light with the exception of the light from the laser.

8. A device for examining surfaces (1) for recording the surface characteristics of a fibrous structured elongated object (3), comprising a transport device (4) with a direction of transport (9) and a transversal direction (10) perpendicular to it for transporting the object (3) in a position at least roughly parallel with a longitudinal extension of the object (3) relative to the transversal direction (10) and at least one device (2) for recording the surface characteristics of a fibrous structured elongated object (3), with a direction of the fibres preferably parallel with the longitudinal extension of the object (3), in which the object (3) is transported in a position in which the longitudinal extension in a transversal direction (10) is angled obliquely to the direction of transport, in which the recording device (2) is made in accordance with any of the foregoing claims, and in which two beams of light (14) side by side, generated by at least one of the light sources (11) follow one another in a direction at least roughly parallel with the transversal direction (10).

9. The device for examining surfaces (1) according to claim 8, **characterised in that** the plane (26), formed by a central position of the light sources (11) which generate the beams of light (14) and by a first point of incidence (20) of a first central ray of light (21) of a first beam of light (16) and a second point of incidence (22) of a second central ray of light (23) of a second beam of light (18), is angled at least roughly perpendicularly to the direction of transport (9).

10. The device for examining surfaces (1) according to claim 8 or 9, **characterised in that** the beams of light (14) are positioned in such a way that two tracks of light (15) side by side overlap relative to the transversal direction (10) or are side by side without gaps relative to the transversal direction (10).

11. The device for examining surfaces (1) according to any of the claims from 8 to 10, **characterised in that** it has a control device (8) and an analysis unit (7).

12. The device for examining surfaces (1) according to any of the claims from 8 to 11, **characterised in that** it has a device (5) for measuring a distance (6), around which the object (3) is fed in the direction of transport (9).

13. A method for recording the surface characteristics of a fibrous structured elongated object (3) in which
there is a transport device (4) with a direction of transport (9) and a transversal direction (10) perpendicular to it for transporting the object (3) in a position at least roughly parallel with a longitudinal extension of the object (3) relative to the transversal direction (10),
there is at least one light source (11) for generating at least two flat beams of light (14), directed on a surface (13) of the object (3), in which the beams of light (14) generated by it, that is to say, the tracks of light (15) it generates and two beams of light (14) side by side follow one another on the surface (13) of the object (3), in a position at least roughly parallel with the transversal direction (10),
there is a camera (12) for recording the tracks of light (15),
there is a device (5) for measuring a distance (6), around which the object (3) is fed in the direction of transport (9),
there is a control device (8) and an analysis unit (7) and
the object (3) is fed through the transport device (4) in the direction of transport (9), in which a sequence of images of the tracks of light (15) and a sequence of distances (6) correlated with the images are recorded, the method being **characterised in that** both of the flat beams of light (14) are generated in such a way that on the same flat surface (13) of the object (3), parallel with the direction of transport (9) and with the transversal direction (10), tracks of light (15) are generated which are angled obliquely along the entire length, both relative to the transversal direction (10) and relative to the direction of transport (9).

14. The method according to claim 13, **characterised in that** the object (3) is fed with a purely translational motion.

15. The method according to claim 13 or 14, **characterised in that** the beams of light (14) are positioned in such a way that two tracks of light (15) side by side overlap relative to the transversal direction (10) or are side by side without gaps relative to the transversal direction (10).

16. The method according to any of the claims from 13 to 15, **characterised in that** the camera (12) is an electronic camera (12), in particular a CMOS camera.

17. The method according to any of the claims from 13 to 16, **characterised in that** only image points from the zones in which tracks of light (15) are present are transferred from the camera (12) to the analysis unit (7).

18. The method according to any of the claims from 13 to 17, **characterised in that** from the sequence of images of the tracks of light (15) the analysis unit (7) generates an image on the surface (13) of the object (3).

## Revendications

1. Dispositif (2) pour l'enregistrement des caractéristiques de la surface d'un objet (3) allongé à structure fibreuse, avec un sens des fibres de préférence parallèle à l'extension longitudinale de l'objet (3), où l'objet (3) est acheminé dans une position, dans laquelle l'extension longitudinale est orientée dans un sens transversal (10) oblique par rapport au sens de son acheminement, qui comprend au moins une source lumineuse (11) qui émet des rayons lumineux et au moins une chambre photographique (12) pour l'enregistrement des rayons lumineux réfléchis par l'objet (3), où une source lumineuse (11) ou les sources lumineuses (11) est structurée ou sont structurées pour générer au moins deux faisceaux de rayons lumineux (14) plats, dirigés sur une surface (13) de l'objet (3) et où un premier faisceau de rayons lumineux (16) et un second faisceau de rayons lumineux (18) sont disposés de manière à ce qu'au point d'incidence des faisceaux des rayons lumineux (14) sur une même surface plane (13) de l'objet (3), parallèlement au sens transversal cité (10) et au sens d'acheminement cité, les deux faisceaux de rayons lumineux (16, 18) génèrent une première trace lumineuse (17) et une seconde trace lumineuse (19) sur la surface (13), **caractérisé en ce que** les traces lumineuses (17, 19) générées suivant le sens transversal (10) sont orientées de manière oblique sur toute leur longueur.

2. Dispositif (2) selon la revendication 1, **caractérisé en ce que** les faisceaux de rayons lumineux (14) présentent respectivement une structure plate.

3. Dispositif (2) selon la revendication 1 ou 2, **caractérisé en ce que** la chambre photographique (12) a été réalisée avec un élément optique (43) et qu'un champ visuel (28) de la chambre photographique (12) dans le sens des droites (24) formées par les points d'incidence (20, 22) voisins des rayons lumineux (21, 23) centraux est agrandi par cet élément optique (43).

4. Dispositif (2) selon une des revendications précédentes, **caractérisé en ce que** la chambre photographique (12) est formée d'une chambre photographique CMOS.

5. Dispositif (2) selon une des revendications précédentes, **caractérisé en ce que** la chambre photographique (12) est munie d'un composant (45) électronique pour la préparation des points image enregistrés ou pour la réduction de la quantité des données à transmettre.

6. Dispositif (2) selon une des revendications précédentes, **caractérisé en ce que** la source lumineuse (11) ou les sources lumineuse (11) est formée ou sont formées d'un laser.

7. Dispositif (2) selon la revendication 6, **caractérisé en ce que**, la chambre photographique (12) est réalisée avec un filtre (44) pour retenir la lumière à l'exception de la lumière du laser.

8. Dispositif pour l'examen des surfaces (1) pour l'enregistrement des caractéristiques de la surface d'un objet (3) allongé à structure fibreuse, qui comprend un dispositif d'acheminement (4) avec un sens d'acheminement (9) et un sens transversal (10) qui lui est perpendiculaire pour l'acheminement de l'objet (3) dans une position pour le moins approximativement parallèle à une extension longitudinale de l'objet (3) par rapport au sens transversal (10) et au moins un dispositif (2) pour l'enregistrement des caractéristiques de la surface d'un objet (3) allongé à structure fibreuse, avec un sens des fibres de préférence parallèle à l'extension longitudinale de l'objet (3), où l'objet (3) est conduit dans une position, dans laquelle l'extension longitudinale en sens transversal (10) est orientée obliquement par rapport au sens du convoyage, où le dispositif (2) cité pour l'enregistrement est réalisé conformément à une quelconque des revendications précédentes, et où deux faisceaux de rayons lumineux (14) respectivement voisins, générés par au moins une des sources lumineuses (11) citées, se suivent dans un sens pour le moins approximativement parallèle par rapport au sens transversal (10).

9. Dispositif pour l'examen des surfaces (1) selon la revendication 8, **caractérisé en ce que** le plan (26), qui est déterminé par une position centrale des sources lumineuses (11) qui génèrent les faisceaux de rayons lumineux (14) et d'un premier point d'incidence (20) d'un premier rayon lumineux (21) central d'un premier faisceau de rayons lumineux (16) et un second point d'incidence (22) d'un second rayon lumineux (23) central d'un second faisceau de rayons lumineux (18), est orienté pour le moins approximativement perpendiculairement par rapport au sens de convoyage (9).

10. Dispositif pour l'examen des surfaces (1) suivant la revendication 8 ou 9, **caractérisé en ce que** les faisceaux de rayons lumineux (14) sont disposés de manière à ce que les deux traces lumineuses (15) respectivement voisines se superposent par rapport au sens transversal (10) ou sont côte à côte sans interstices par rapport au sens transversal (10).

11. Dispositif pour l'examen des surfaces (1) suivant une des revendications de 8 à 10, **caractérisé en ce qu'**il présente un dispositif de commande (8) et une unité d'analyse (7).

12. Dispositif pour l'examen des surfaces (1) suivant une des revendications de 8 à 11, **caractérisé en ce qu'**il présente un dispositif (5) pour la mesure d'une distance (6), autour duquel l'objet (3) avance dans le sens d'acheminement (9).

13. Procédé pour l'enregistrement des caractéristiques de la surface d'un objet (3) allongé à structure fibreuse dans lequel
est logé un dispositif d'acheminement (4) avec un sens d'acheminement (9) et un sens transversal (10) perpendiculaire au premier pour l'acheminement de l'objet (3) dans une position pour le moins approximativement parallèle à une extension longitudinale de l'objet (3) par rapport au sens transversal (10),
est logée au moins une source lumineuse (11) pour générer au moins deux faisceaux de rayons lumineux (14) plats, dirigés sur une surface (13) de l'objet (3), où les faisceaux des rayons lumineux (14) générés par celle-ci ou les traces lumineuses (15) générées par celle-ci et deux faisceaux de rayons lumineux (14) respectivement voisins se suivent sur la surface (13) de l'objet (3), dans une position pour le moins approximativement parallèle par rapport au sens transversal (10),
est logée une chambre photographique (12) pour l'enregistrement des traces lumineuses (15),
est logé un dispositif (5) pour la mesure d'une distance (6), autour de laquelle l'objet (3) avance dans le sens d'acheminement (9),
sont logés un dispositif de commande (8) et une unité d'analyse (7) et
l'objet (3) est conduit au moyen d'un dispositif d'acheminement (4) dans le sens d'acheminement (9), où sont enregistrées une séquence d'images des traces lumineuses (15) et une séquence de distances (6) liées aux images, **caractérisé en ce que** les deux faisceaux de rayons lumineux (14) plats sont générés de manière à ce que sur une même surface plane (13) de l'objet (3), parallèlement au sens d'acheminement cité (9) et au sens transversal cité (10), des traces lumineuses (15) sont générées, orientées obliquement tout au long de leur longueur, que ce soit par rapport au sens transversal (10) ou par rapport au sens de convoyage (9).

14. Procédé selon la revendication 13, **caractérisé en ce que** l'objet (3) est acheminé suivant un mouvement strictement translatoire.

15. Procédé selon la revendication 13 ou 14, **caractérisé en ce que** les faisceaux des rayons lumineux (14) sont disposés de manière à ce que les deux traces lumineuses (15) respectivement voisines se superposent par rapport au sens transversal (10) ou se superposent sans interstices par rapport au sens transversal (10).

16. Procédé selon une des revendications de 13 à 15, **caractérisé en ce qu'**il est utilisé comme chambre photographique (12) une chambre photographique électronique (12), plus précisément une chambre photographique CMOS.

17. Procédé selon une des revendications de 13 à 16, **caractérisé en ce que**, de la chambre photographique (12), ne sont transférées à l'unité d'analyse (7) que les points image des zones dans lesquelles des traces lumineuses (15) sont présentes.

18. Procédé selon une des revendications de 13 à 17, **caractérisé en ce que** l'unité d'analyse (7), à partir de la séquence d'images des traces lumineuses (15), génère une image sur la surface (13) de l'objet (3).
